# EUROPEAN PATENT SPECIFICATION

(11) **EP 2 092 959 B1**
(45) Date of publication and mention of the grant of the patent: **27.06.2012**
(21) Application number: 09159796.3
(22) Date of filing: 26.01.2006
(51) Int. Cl.: A61N 1/37, A61N 1/362, A61N 1/368

(54) **Anodal capture**
Anodische Erfassung
Capture anodique

(43) Date of publication of application: 26.08.2009
(62) Divisional of application: 06701334.2
(73) Proprietor: St. Jude Medical AB, 175 84 Järfälla (SE)
(72) Inventor: Holmström, Nils, SE-175 49, Järfälla (SE); Björling, Anders, SE-169 37, Solna (SE)

(56) References cited:
- WO-A-2009/020639
- US-A1- 2002 082 651
- US-B1- 6 687 545
- US-B1- 7 389 140
- THIBAULT B ET AL: "Anodal right ventricular capture during left ventricular stimulation in CRT-implantable cardioverter defibrillators" PACE - PACING AND CLINICAL ELECTROPHYSIOLOGY, BLACWELL FUTURA PUBLISHING, MALDEN, MA, US, vol. 28, no. 7, 1 July 2005 (2005-07-01), pages 613-619, XP003000370 ISSN: 0147-8389

## Description

### Field of the invention

The present invention relates to a device according to the preamble of the independent claim, and in particular to a biventricular implantable heart stimulating device where left ventricular (LV) stimulation is performed between an LV-tip electrode, being the cathode, and a right ventricular (RV) ring electrode, being the anode.

### Background of the invention

When stimulating LV-tip to RV-ring in a biventricular system a so called anodal stimulation generating an anodal capture may occur on the RV-ring. If the left ventricle is stimulated first - which it often is - both ventricles will depolarize at the same time and a ventricle-ventricle (VV) delay optimization is then not possible to perform.

Furthermore, an automatic capture algorithm may detect loss of capture at each RV stimulation since the RV has already been stimulated and is thus refractory. This, in turn, will lead to unnecessary going into high output mode and incorrect diagnostics.

As will be discussed in detail in the following the above is related to that the unipolar voltage strength-duration curves for the LV tip and the RV ring electrodes have different shapes. Anodal thresholds are normally higher than cathodal thresholds for the same electrode. The LV thresholds are normally higher than RV thresholds and the ring thresholds are normally higher than the tip thresholds because of different surface area and distance to excitable tissue.

All these circumstances influence the bifocal stimulation thresholds so that anodal threshold may be higher than cathodal at wide pulse width, while the cathodal threshold may be higher for a short pulse width.

In order to fully explain the present invention a general background will be given in the following.

In order to excite the left ventricle, the lead must be disposed near the left ventricle, preferably in the region of the free lateral or posterior wall, which may most easily be accomplished by placing the lead through the coronary sinus and into a left cardiac vein. Unlike a lead for the right ventricle, which is disposed within the ventricle where a tip electrode can be fixed into the myocardium, the electrodes of a lead in a cardiac vein cannot be fixed into the myocardium since that would require puncturing the vein. Instead, in the case of a bipolar lead, both the tip and ring electrodes (or proximal and distal electrodes in the case where both electrodes are ring electrodes or other types of structures) are positioned within the vein adjacent to the left ventricular myocardium. Because the surface area of the tip electrode is smaller than the area of the ring electrode, the current density will be higher at the tip electrode and, thus, the threshold lower. Normally, therefore, the tip of a bipolar lead is used as the cathode in order to achieve the desirable cathodal capture when a voltage pulse is impressed across the two electrodes. (Cathodal capture means that cathodal stimulation is responsible for the contraction.) With a bipolar lead in a cardiac vein, on the other hand, both electrodes are external to the myocardium and may have similar capture thresholds so that either anodal or cathodal capture can occur when a pacing pulse is output through the lead. A problem arises when the pulse energy for a bipolar lead in a cardiac vein is adjusted. When the lead is implanted, the capture threshold for the tip or distal electrode (i.e., the electrode usually selected to function as the cathode) may be higher than that of the ring or proximal electrode. When the clinician then determines the capture threshold of the lead with a bipolar pulse in order to adjust the stimulus pulse energy, it is impossible to distinguish between anodal and cathodal capture. There is then a risk that the stimulus pulse energy will be set to an anodal capture threshold when the cathodal capture threshold is higher. As the anodal capture threshold increases over time, the stimulus pulses may no longer be of sufficient energy to excite the left ventricle (diminishing or eliminating the programmed safety margin), and the patient may experience sporadic or total loss of resynchronization therapy.

Thibault et al. ("Anodal right ventricular capture during left ventricular stimulation in CRT-implantable cardioverter defibrillators", PACE, vol. 28, 2005, pp. 613-619) disclose an implantable heart stimulating device containing all the features of the preamble of claim 1.

US-6,687,545 relates to a cardiac stimulation system and method for performing automatic capture verification during bipolar stimulation by eliminating capture verification during a cardiac cycle in which anodal stimulation is detected. Anodal stimulation is detected by the absence of a delay between the bipolar stimulation pulse and an evoked response sensed at the electrode functioning as the anode during stimulation.

Automatic capture verification during bipolar stimulation is recommended only if anodal stimulation is not detected at a working stimulation output. During automatic capture verification, if anodal stimulation is detected, a capture threshold search is performed.

In US-6,687,545 unipolar sensing is performed using e.g. the right ventricular ring electrode and the housing to determine if a stimulation pulse produced anodal stimulation at the ring electrode.

According to the US-patent this is performed by determining the time from the stimulation pulse to the onset of the evoked response.

Typically, a 20 to 40 ms conduction delay to the unipolar ring evoked response signal occurs when only cathodal stimulation is present. Therefore, if there is a delay to the evoked response as determined then anodal stimulation is not indicated and will not interfere with evoked response detection during bipolar evoked response sensing of the bipolar stimulation at the currently programmed output.

If no delay to the evoked response is measured then anodal stimulation is occurring at the ring electrode at the programmed stimulation output.

Thus, the system and method disclosed in US-6,687,545 may be used to verify anodal stimulation.

US-6,421,564 relates to a bi-chamber pacing system employing unipolar left heart chamber lead in combination with bipolar right chamber lead. The object is to achieve a system where the left ventricle pacing pulse in a left ventricular pacing vector is directed such that the vector traverses as great a bulk of the left ventricular myocardial mass as possible.

US-6,611,712 relates to an apparatus and method for testing the capture threshold of a bipolar lead of a cardiac rhythm management device in order to determine an appropriate stimulus pulse energy for the lead and/or select an appropriate stimulation configuration. The inventor of the present invention has identified two main reasons for the stimulation set-up where the stimulation pulse is applied between a left ventricular (LV) coronary sinus (CS) lead tip electrode, being the cathode, and a right ventricular (RV) ring electrode.

The first reason is to avoid stimulation of nervus phrenicus.

The tip electrode of an LV CS electrode lead is often positioned in close proximity of the nervus phrenicus, which is a nerve that controls the contraction of the diaphragm. It has been found that a direction of the electrical stimulation vector, resulting from a stimulation pulse from an electrode close to nervus phrenicus, that essentially encompasses the nerve, may result in a nerve stimulation that in turn may cause diaphragm contractions. That may especially occur when a bipolar LV CS electrode lead is used, i.e. a ring electrode of the LV CS lead as indifferent electrode (anode).

That is one reason of instead using the ring electrode of an RV electrode lead in that the electrical stimulation vector then is directed away from nervus phrenicus thus avoiding stimulated diaphragm contractions.

Another reason is that if unipolar stimulation is applied using the housing (case) of the implantable device as indifferent electrode to a LV CS tip electrode, unwanted pocket stimulation may occur, i.e. anodal stimulation at the indifferent case electrode.

Thus, the object of the present invention is to handle the situation occurring when anodal stimulation is detected at an RV ring electrode during a biventricular stimulation mode between an LV CS lead electrode and an RV lead electrode in order to secure safe and reliable performance of the implantable stimulation device.

### Summary of the invention

The above-mentioned object is achieved by the present invention according to the independent claim.

Preferred embodiments are set forth in the dependent claims.

### Short description of the appended drawings

Figure 1 illustrates two graphs that show strength duration curves in two different situations.
Figure 2 is a block diagram illustrating a preferred embodiment of the present invention.

### Detailed description of preferred embodiments of the invention

As stated above the present invention concerns an implantable heart stimulating device, in particular a biventricular pacemaker system, where LV stimulation is performed between the LV-tip (cathode) and the RV-ring (anode).

In a bi-ventricular pacing system, a small diameter, often unipolar, left ventricular (LV), coronary sinus (CS) electrode lead and a bipolar right ventricular (RV) endocardial electrode lead are preferably employed to provide left and right heart chamber pacing/sensing electrodes. The LV CS lead is advanced through the superior vena cava, the right atrium, the ostium of the coronary sinus (CS), the CS, and into the coronary vein descending from the CS to locate the LV active pace/sense electrode at a desired LV pace/sense site.

The RV electrode lead is advanced into the RV chamber to locate RV tip and ring electrodes therein.

A requirement that makes non-simultaneous biventricular pacing and VV-delay optimization is that no anodal ring stimulation capturing the RV-ring is present.

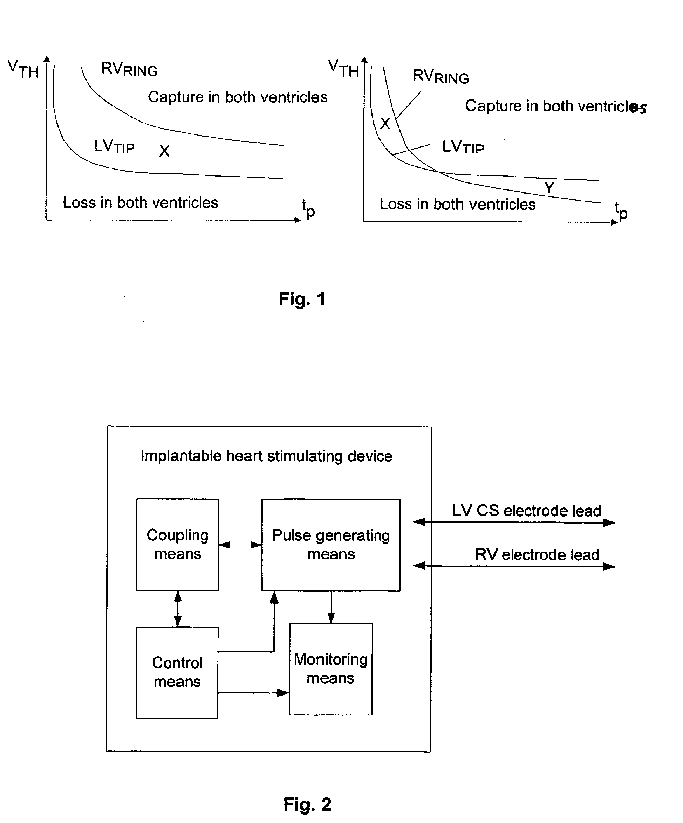

Thus, and according to an example if anodal RV-ring stimulation is detected the LV pulse width is preferably adjusted so that LV-tip capture is obtained while anodal ring stimulation is not capturing the RV. This is schematically illustrated in figure 1 (see area "X" in figure 1).

Figure 1 illustrates examples of unipolar strength-duration curves at anodal RV ring stimulation and cathodal LV tip stimulation. The Y-axis designates the stimulation threshold in volts, i.e. the amplitude of a stimulation pulse, and the X-axis designates the pulse width.

In the area "X" there is loss of capture in the right ventricle but capture in the left ventricle.

With references to the left graph there is loss of capture in both ventricles for stimulation pulses having the amplitudes and pulse widths below both curves, and capture in both ventricles above both curves. This graph illustrate a normal situation where the stimulation threshold is much higher on the anode (RV ring) than on the cathode (LV tip).

The right graph illustrates a situation where the two strength-duration curves are close to each other or even cross. In the area "Y" there is capture in the right ventricle but loss in the left ventricle. In "Y" it is not possible to only get capture in the left ventricle.

If the thresholds in RV and LV are close to each other (figure 1, right graph), it is, however, recommended not to use the RV ring as anode for the stimulation current. Instead the RV-ring in combination with the SVC-coil, RV-coil and/or the case is better to use. This is in accordance with a preferred embodiment of the present invention, which will be further discussed below.

There are many different ways of detecting anodal (or bifocal) stimulation.

The above-mentioned US-6,687,545 discloses a device where anodal stimulation is monitored by measuring the delay of the evoked response at the ring electrode, and if no delay is detected anodal stimulation is considered detected.

Other indications for anodal RV stimulation are:

### Always "loss" at RV-stimulation.

Normally the left ventricle is stimulated before the right ventricle. Beat-to-beat capture verification is performed in the right ventricle being the last ventricle. If the system cannot detect capture in the last ventricle, despite the threshold searches postulate that it should be, it is an indication of anodal stimulation in the right ventricle before the actual RV stimulation. The reason for non-capture is that the myocardium in the right ventricle is refractory at the time of the RV stimulation due to bifocal capture at the time of the first stimulation in the left ventricle.

### Always "capture" at RV-stimulation.

Again, normally the left ventricle is stimulated before the right ventricle. If the VV-delay is short an anodal RV-ring capture may be detected as capture from a stimulation pulse applied to the RV-ring. In this case the right ventricle heart tissue is refractory resulting in non-capture irrespectively of the level of the stimulation amplitude applied to the RV-ring electrode, because it is the LV stimulation pulse that stimulates the RV. Thus, if we still get capture in the RV (being the last ventricle) with zero pulse amplitude at the RV ring electrode it is an indication of anodal RV stimulation.

The threshold searches of the electrodes may be performed by temporarily switch mode to RV pacing (LV off). Then a threshold search with an anodal pacing pulse (e.g. the pacing may be between the case (negative) and the RV-ring (positive)) which will reveal the actual anodal threshold of the RV-ring. The same procedure is then performed in the LV (RV off) but with cathodic pacing pulse. If the two threshold values are close or even lower in the RV it is clear that it is difficult to find a stimulation pulse characteristic that avoids anodal stimulation of the RV-ring, instead other combinations of indifferent electrodes may be used according to the preferred embodiment.

Still another way to detect anodal RV-ring stimulation is to analyze the IEGM in the RV at biventricular stimulation with the LV before the RV. By studying the evoked response (ER) morphology in the RV and changing the output level in the LV it is possible to find a point where the ER shifts in time from starting at LV-stim to RV-stim. A morphology change is likely to occur at this point. An obvious limitation with this method is that the RV threshold must be higher than the LV threshold.

When anodal ring stimulation in RV is detected and verified, actions have to be taken.

According to the example threshold searches are performed at different pulse widths to find the optimal pulse width that gives the highest difference in cathodal LV threshold and anodal RV threshold. In this point the anodal RV strength duration curve must be above the cathodal LV threshold.

With references to figure 2, showing a block diagram of the inventive implantable heart stimulating device, the present invention will be further described.

The implantable heart stimulating device, according to the example, comprises a left ventricular (LV) coronary sinus (CS) electrode lead at least provided with a tip electrode, a right ventricular (RV) electrode lead at least provided with a ring electrode, a pulse generating means connected to the leads and adapted to apply a stimulation pulse between the tip electrode and ring electrode, with the tip electrode being the cathode. The device also comprises a monitoring means adapted to monitor for and detect anodal stimulation at the right ventricular ring electrode subsequent to a stimulation. The monitoring means includes features necessary to perform normal evoked response detection. If anodal stimulation at the right ventricular ring electrode is detected a threshold search is performed by varying the pulse width and/or pulse amplitude in order to identify stimulation pulse characteristics that avoid anodal stimulation at the ring electrode.

In addition the device comprises a control means connected to the monitoring means and to the pulse generating means. In response of a detected anodal stimulation the control means generates and applies control signals to the pulse generating means in order to initiate the threshold search.

As a result of the performed threshold search or searches, strength duration curves of the electrode(s) may be established and used to identify the stimulation pulse characteristics that avoid anodal stimulation at the ring electrode. In that case the identified stimulation pulse characteristics are chosen such that they are above the strength duration curve of the LV CS tip electrode but below the strength duration curve of the RV ring electrode, when inserted into a strength duration curve diagram (cf. the "X"-area in the left graph of figure 1).

According to the preferred embodiment, if anodal RV stimulation is detected, a change or a proposal for a change is made of the anode for LV pacing from RV-ring to another electrode configuration. This electrode configuration includes the RV-ring and one or many of the case, RA-ring, LV-ring, RV-coil or SVC-coil (if available).

Also here referring to figure 2 the implantable heart stimulating device comprises a left ventricular (LV) coronary sinus (CS) electrode lead at least provided with a tip electrode, a right ventricular (RV) electrode lead at least provided with a ring electrode, a pulse generating means connected to the leads and adapted to apply a stimulation pulse between the tip electrode and ring electrode, with the tip electrode being the cathode. As in the example the device comprises a monitoring means adapted to monitor for and detect anodal stimulation at said right ventricular ring electrode subsequent to a stimulation. The monitoring means includes features necessary to perform normal evoked response detection.

If anodal stimulation at the right ventricular ring electrode is detected, at least one further electrode is arranged to function as indifferent electrode together with the ring electrode. This is achieved by electrically connecting the ring electrode to the at least one further electrode by a coupling means in response of control signals generated by a control means.

The further electrode is arranged at any of the leads and may e.g. be a coil electrode at the right or left ventricular electrode lead and/or at the housing of the device.

Thus, the monitoring of anodal stimulation may e.g. be performed by measuring the delay of the evoked response at the ring electrode, and if no delay is detected anodal stimulation is considered detected. This is further described above in connection with the prior art document US-6,687,545.

Other ways of monitoring and detecting anodal stimulation are described above.

In the following some other aspects of anodal stimulation are briefly discussed.

If the anodal RV-ring threshold is lower than the LV threshold and the patient benefits from a short VV-delay the RV stimulation can just be turned off. Instead every ventricular stimulation will stimulate both LV and RV simultaneously which saves energy. Evoked response detectors are still active in both ventricles to verify capture. If a loss occurs in either ventricle the pacing amplitude is increased as usual. If that does not help to get anodal capture in RV the system switches the pacing mode to dual ventricular pulse (preferably not with RV ring as anode).

The actions mentioned above can either be performed automatically by the device or be performed by the healthcare personnel at next follow up at the healthcare centre. The alert to change setting may be communicated to the physician via the programmer.

If anodal stimulation suddenly appears it is recommended to perform threshold search also in the right ventricle since the cathodic threshold may also have changed.

The present invention is not limited to the above-described preferred embodiment. Various alternatives, modifications and equivalents may be used. Therefore, the above embodiment should not be taken as limiting the scope of the invention, which is defined by the appending claims.

## Claims

1. Implantable heart stimulating device comprising a left ventricular (LV) coronary sinus (CS) electrode lead at least provided with a tip electrode, a right ventricular (RV) electrode lead at least provided with a ring electrode, a pulse generating means connected to said leads and adapted to apply a stimulation pulse between said tip electrode and ring electrode, with the tip electrode being the cathode,
**characterized in that** the device comprises a monitoring means adapted to monitor for and detect anodal capture at said right ventricular ring electrode subsequent to a stimulation, and if anodal capture at said right ventricular ring electrode is detected, to arrange at least one further electrode to function as indifferent electrode together with the ring electrode.

2. Implantable heart stimulating device according to claim 1, wherein said further electrode is arranged at any of the leads.

3. Implantable heart stimulating device according to claim 1, wherein said further electrode is arranged at the housing of the device.

4. Implantable heart stimulating device according to claim 1, wherein said further electrode is a coil electrode arranged at the right ventricular electrode lead.

5. Implantable heart stimulating device according to anyone of preceding claims, wherein the ring electrode is electrically connectable to said further electrode(s), by a coupling means in response of control signals generated by a control means.

6. Implantable heart stimulating device according to any one of preceding claims, wherein anodal stimulation is monitored by measuring the delay of the evoked response at the ring electrode, and if no delay is detected anodal stimulation is considered detected.

## Patentansprüche

1. Implantierbare Herzstimulationsvorrichtung, die eine linksventrikuläre (LV) Koronarsinus(KS)-Elektrodenleitung umfasst, die mindestens mit einer Spitzenelektrode versehen ist, eine rechtventrikuläre (RV) Elektrodenleitung, die mindestens mit einer Ringelektrode versehen ist, ein Impulserzeugungsmittel, das mit den Leitungen verbunden und angepasst ist, zwischen der Spitzenelektrode und Ringelektrode einen Stimulationsimpuls abzugeben, wobei die Spitzenelektrode die Kathode ist,
**dadurch gekennzeichnet, dass** die Vorrichtung ein Überwachungsmittel umfasst, das angepasst ist, auf die anodale Erregungsauslösung an der rechtsventrikulären Ringelektrode im Anschluss an eine Stimulation hin zu überwachen und sie zu erfassen, und wenn die anodale Erregungsauslösung an der rechtsventrikulären Ringelektrode erfasst wird, dafür zu sorgen, dass mindestens eine weitere Elektrode als indifferente Elektrode zusammen mit der Ringelektrode fungiert.

2. Implantierbare Herzstimulationsvorrichtung nach Anspruch 1, wobei die weitere Elektrode an einer der Leitungen angeordnet ist.

3. Implantierbare Herzstimulationsvorrichtung nach Anspruch 1, wobei die weitere Elektrode am Gehäuse der Vorrichtung angeordnet ist.

4. Implantierbare Herzstimulationsvorrichtung nach Anspruch 1, wobei die weitere Elektrode eine Wendelelektrode ist, die an der rechtsventrikulären Elektrodenleitung angeordnet ist.

5. Implantierbare Herzstimulationsvorrichtung nach einem der vorhergehenden Ansprüche, wobei die Ringelektrode über ein Kopplungsmittel als Reaktion auf Steuersignale, die von einem Steuermittel erzeugt werden, elektrisch mit der bzw. den weiteren Elektrode(n) verbunden werden kann.

6. Implantierbare Herzstimulationsvorrichtung nach einem der vorhergehenden Ansprüche, wobei die anodale Stimulation überwacht wird, indem die Verzögerung der Reizantwort an der Ringelektrode gemessen wird, und wenn keine Verzögerung erfasst wird, die anodale Stimulation als erfasst gilt.

## Revendications

1. Dispositif de stimulation cardiaque implantable comprenant un conducteur d'électrode du sinus coronaire (CS) ventriculaire gauche (LV) doté au moins d'une électrode à pointe, un conducteur d'électrode ventriculaire droite (RV) doté au moins d'une électrode annulaire, un moyen de génération d'impulsions connecté auxdits conducteurs et adapté pour appliquer une impulsion de stimulation entre lesdites électrode à pointe et électrode annulaire, avec l'électrode à pointe étant la cathode,
**caractérisé en ce que** le dispositif comprend un moyen de surveillance adapté pour surveiller et détecter une capture anodique au niveau de ladite électrode annulaire ventriculaire droite à la suite d'une stimulation, et si une capture anodique est détectée au niveau de ladite électrode annulaire ventriculaire droite, pour faire en sorte qu'au moins une autre électrode fonctionne en tant qu'électrode indifférente avec l'électrode annulaire.

2. Dispositif de stimulation cardiaque implantable selon la revendication 1, dans lequel ladite autre électrode est disposée sur n'importe lequel des conducteurs.

3. Dispositif de stimulation cardiaque implantable selon la revendication 1, dans lequel ladite autre électrode est disposée sur le boîtier du dispositif.

4. Dispositif de stimulation cardiaque implantable selon la revendication 1, dans lequel ladite autre électrode est une électrode enroulée disposée sur le conducteur d'électrode ventriculaire droite.

5. Dispositif de stimulation cardiaque implantable selon l'une quelconque des revendications précédentes, dans lequel l'électrode annulaire peut être connectée électriquement à ladite / auxdites autre(s) électrode(s) par un moyen d'accouplement en réaction à des signaux de contrôle générés par un moyen de contrôle.

6. Dispositif de stimulation cardiaque implantable selon l'une quelconque des revendications précédentes, dans lequel la stimulation anodique est surveillée en mesurant le délai de la réaction suscitée au niveau de l'électrode annulaire, et si aucun délai n'est détecté, la stimulation anodique est considérée comme détectée.
